# EUROPÄISCHE PATENTSCHRIFT

(11) **EP 1 348 393 B1**
(45) Veröffentlichungstag und Bekanntmachung des Hinweises auf die Patenterteilung: **21.03.2007**
(21) Anmeldenummer: 02007218.7
(22) Anmeldetag: 27.03.2002
(51) Int. Cl.: A61B 19/00

(54) **Medizinische Navigation bzw. prä-operative Behandlungsplanung mit Unterstützung durch generische Patientendaten**
Medical navigation or pre-operative treatment planning supported by generic patient data
Procédé de navigation ou planification de traitement pré-opératoire assisté par données de patients génériques

(43) Veröffentlichungstag der Anmeldung: 01.10.2003
(73) Patentinhaber: BrainLAB AG, 85551 Kirchheim/Heimstetten (DE)
(72) Erfinder: Vilsmeier, Stefan, 6330 Kufstein (AT)
(74) Vertreter: Schwabe - Sandmair - Marx

(56) Entgegenhaltungen:
- WO-A-99/59106
- DE-A- 10 037 491
- US-A- 6 033 415
- "Facial Analysis in Two and Three Dimensions", Radiographic Cephalometrics, Richard L. Jacobson, Chapter 21

## Beschreibung

Die vorliegende Erfindung betrifft ein Verfahren zur computergestützten, medizinischen Navigation bzw. zur prä-operativen Behandlungsplanung. Ganz allgemein werden bei solchen Navigationsverfahren mittels einer Positionserfassungseinheit die aktuelle Position eines Patienten oder eines Patientenkörperteils sowie die Positionen von medizinischen Behandlungsgeräten oder behandlungsunterstützenden Geräten erfasst, und die erfassten Positionsdaten werden Körperstrukturdaten zugeordnet, um die Körperstrukturdaten in Zuordnung zu den Positionsdaten gemeinsam im Rahmen der Behandlungsunterstützung zu verwenden. Ein solches Navigationssystem ist beispielsweise in der DE 196 39 615 C2 beschrieben.

Computergestützte, stereotaktische Systeme, die mit Hilfe von Körperstrukturdaten arbeiten, welche aus Tomographie-Erfassungssystemen erhalten wurden, und welche mit Unterstützung von vor Ort erstellten Röntgenbildern arbeiten, sind beispielweise aus der US 4,791,934 und der US 5,799,055 bekannt. Operationsunterstützung durch Röntgenbildaufnahmen wird wiederum beispielsweise in den US-Patenten 5,967,982; 5,772,594 und 5,784,431 erörtert.

Wenn eine genau arbeitende medizinische Navigation zur Verfügung gestellt werden soll, wird gemäß dem derzeitigen Stand der Technik noch immer unter Zuhilfenahmen von Körperstrukturdaten gearbeitet, die zum Beispiel aus Schichtbild-Erfassungssystemen stammen, wie zum Beispiel Computertomographiegeräten oder Kernspintomographiegeräten. Der zu behandelnde Patient wird dabei vor Ort positionell gegenüber den vorher ermittelten Bilddaten registriert, und Operationsinstrumente werden dann virtuell zu den Bilddaten in gleicher Relation wie zum realen Patienten dargestellt, um die Körperstrukturdaten oder möglicherweise auch Röntgenbilddaten für den Chirurgen im Operationsraum nutzbar zu machen.

Der Nachteil solcher Verfahren, bei denen extra für die Navigation im Rahmen einer Behandlung Schichtbildaufnahmen (CT, MR) oder Röntgenbilder erstellt werden, liegt einerseits in der Strahlenbelastung für den Patienten, die dabei entsteht, und andererseits in dem hohen Kostenaufwand, da solche Geräte sowohl in der Anschaffung als auch in der Wartung und im Betrieb sehr teuer sind.

Es ist versucht worden, Systeme zu entwickeln, die sich ohne vorab separat erfasste Körperstrukturdaten des Patienten einsetzen lassen, beispielsweise auf der Basis von statistischen Modellen von Bilddatensätzen für Körperstrukturen. Jedoch mangelt es solchen Systemen an der erforderlichen Genauigkeit für den jeweils zu behandelnden Patienten.

Aus der WO 99/59106 ist ein System zum Zuordnen von Bildern und zum Erzeugen eines 3D-Modells bekannt, wobei ein sogenanntes Sculptor-Modul verwendet wird, um zum Beispiel optisch oder durch Röntgenaufnahmen erzeugte Bilder räumlich zuzuordnen. Mit Hilfe dieses Sculptor-Moduls kann ein Benutzer die Stelle von verschiedenen anatomischen Punkten in jedem der Bilder identifizieren. Hierzu müssen mehrere Ansichten eines Objektes aus verschiedenen Richtungen ausgewertet werden, um vollständig die 3D-Koordinaten (x, y, z) einer bestimmten Landmarke zu bestimmen. Ein so genanntes "stock model" wird gemorpht, um ein patientenspezifisches Modell zu erhalten. Beispielsweise müssen für eine kieferorthopädische Anwendung 200 solcher Landmarken erfasst werden.

Es ist die Aufgabe der vorliegenden Erfindung, ein Verfahren zur computergestützten, medizinischen Navigation bzw. prä-operativen Behandlungsplanung bereitzustellen, welches die oben beschriebenen Nachteile des Standes der Technik überwindet. Insbesondere soll die gesundheitsbelastende und kostenintensive Erstellung eines separaten Bilddatensatzes für die Navigation/Behandlungsplanung vermieden werden, wobei trotzdem eine ausreichend genaue Navigation zur Verfügung gestellt werden soll.

Diese Aufgabe wird durch ein Verfahren nach Anspruch 1 gelöst.

Wenn im Weiteren von "Navigation" die Rede ist, so soll dieser Begriff auch grundsätzlich die prä-operative Behandlungsplanung mit umfassen, im Rahmen der ein Chirurg vorab eine ideale Vorgehensweise für eine Behandlung bestimmt, also beispielsweise die ideale Position eines Implantats manuell bestimmt und festlegt.

Die Vorteile der vorliegenden Erfindung beruhen darauf, dass es mit der Verwendung eines an den Patienten angepassten generischen Modells nicht mehr notwendig ist, für die Behandlung, bei der eine medizinische Navigation bereitgestellt werden soll, einen separaten Datensatz für die Körperstruktur zu erstellen. Einerseits erspart man dem Patienten dabei Strahlungsbelastungen, und die Kosten für solche Datensatzerstellungen (zum Beispiel durch Tomographie), können eingespart werden, während andererseits durch die Verknüpfung der generischen Körperstrukturdaten mit patientencharakteristischen Erfassungsdaten, indem digital rekonstruierte Röntgenbilder des generischen Modells mit Röntgenbilddaten des Patienten in Deckung gebracht werden, um modifizierte Körperstrukturdaten zu erhalten, ein Datensatz zur Verfügung gestellt wird, mit dessen Hilfe eine sehr genaue medizinische Navigation ermöglicht wird. Das generische Modell, das eine Art allgemeingültiges Modell für die betreffende Körperstruktur sein kann, für das sämtliche relevante Daten vorliegen, umfasst dabei zwar nicht Daten, die speziell auf den betreffenden Patienten zugeschnitten sind, jedoch umfasst es ausreichend Anatomie- bzw. Körperstrukturdaten, um nach der Anpassung mit Hilfe patientencharakteristischer Erfassungsdaten eine ausreichend genaue Basis für eine medizinische Navigation zur Verfügung stellen zu können.

Die Unteransprüche definieren bevorzugte Ausführungsformen der Erfindung, wie sie im Folgenden auch spezieller erörtert werden.

Es ist im Rahmen der vorliegenden Erfindung möglich, die Körperstrukturdaten in der Form eines Bilddatensatzes bereitzustellen, insbesondere als Schichtbilderfassungs-Datensatz.

Hierbei werden nicht, wie bei Verfahren gemäß dem Stand der Technik separate Bilderfassungs-Datensätze erzeugt, sondern das generische Modell selbst wird schon in Form eines Bilddatensatzes bereitgestellt, der dann noch an den jeweiligen Patienten angepasst wird, um modifizierte Körperstrukturdaten eines generischen Modells als einen für den Patienten geltenden Bilddatensatz zu erhalten. Mit diesem Bilddatensatz kann dann ebenso gearbeitet werden, wie mit einem, der kostenintensiv und unter Bestrahlungsbelastung präoperativ von dem Patienten erstellt worden ist. Es ist beispielsweise denkbar, ein generisches Modell zu verwenden, das eine typische bzw. durchschnittliche Körperstruktur umfasst, beispielsweise eine einfache modellhafte Darstellung eines Wirbelkörpers oder einer Knochen-/Körperstruktur.

Das generische Modell kann auch ein statistisches Modell der Körperstruktur umfassen, insbesondere basierend auf statistischen Auswertungen einer unbestimmten Anzahl von Bilddatensätzen, zum Beispiel von realen Wirbelkörper-Bilddatensätzen.

Ferner besteht die Möglichkeit, das generische Modell gleich als eine Art Modell-Paket für eine Vielzahl von Körperstrukturen gleicher Art bereitzustellen. In einem solchen Fall ist es bei der Anpassung des Modells möglich, sich aus der Vielzahl der Modelle im Paket dasjenige auszusuchen, welches am besten zu den Röntgenbilddaten des Patienten passt, so dass nur eine geringere computergestützte Anpassung des Modells vorgenommen werden muss.

Das generische Modell umfasst im Rahmen der vorliegenden Erfindung einen dreidimensionalen Datensatz einer Körperstruktur, insbesondere auch ein geometrisches Modell. Mit anderen Worten kann es sich beim generischen Modell um dreidimensionale (zum Beispiel Wirbelkörpermodell) Daten oder aber auch um ein Modell in Form von geometrischen Daten handeln. Diese Daten können beispielsweise Winkel und/oder Trajektorieninformationen sein, die für den Arzt dargestellt werden und ihm beispielsweise die Idealposition eines Implantates anzeigen.

Patientencharakteristische Daten, wie sie zur Anpassung des generischen Modells verwendet werden können, die im Weiteren als Diagnosedaten bezeichnet werden, sind Röntgenbilddaten, und zwar solche aus vorab oder während der Behandlung erstellten Röntgenbildern, insbesondere zwei- oder multiplanare Röntgenbilder. Ein Beispielsfall wäre derjenige, wo für den Patienten schon Röntgenbilder vorliegen, die im Rahmen zeitlich zurückliegender Untersuchungen erstellt worden sind. Daten über Körperstrukturen aus diesen "alten" Röntgenbildern eignen sich insbesondere, wenn Formabweichungen gegenüber dem generischen Modell einberechnet werden sollen.

Es ist aber auch möglich, noch während der Behandlung einzelne Röntgenbilder des Patienten zu erstellen und diese Informationen dann in die Anpassung des generischen Modells einfließen zu lassen. Der Vorteil gegenüber der herkömmlichen "Röntgennavigation" liegt dabei darin, dass nicht eine große Vielzahl von Röntgenbildern erstellt werden muss, wie sie bei der Navigation auf der Basis von Röntgenbildern verwendet wird; es genügt die Erstellung nur eines oder sehr weniger Röntgenbilder zur Anpassung des generischen Modells, die sich außerdem auf einen sehr kleinen Körperabschnitt begrenzen kann. Damit wird die Strahlenbelastung gegenüber herkömmlicher Röntgennavigation stark verringert.

Obiges gilt in gleicher Weise auch für Computertomographie-Bilddaten. Es können solche verwendet werden, die aus sehr viel früher erstellten Tomographieerfassungen hervorgehen, deren Information jedoch genügt, um eine geeignete Anpassung des generischen Modells vorzunehmen.

Die Röntgenbilddaten können außerdem digital rekonstruierte Röntgenbilddaten (DRRs = digitally reconstructed radiographs) sein, die zum Beispiel aus schon vorhandenen Tomographie-Bilddatensätzen erstellt werden können, ohne dass der Patient nochmals einer Röntgenaufnahme unterzogen werden muss.

Die Anpassung des generischen Modells kann im Rahmen der Erfindung durch eine oder mehrere der folgenden Methoden erfolgen:
- manuelle Anpassung mit Hilfe einer Bilddarstellungsunterstützung, insbesondere durch das Versetzen von Punkten und Landmarken oder das Verschieben, Verdrehen, Dehnen, oder Komprimieren des generischen Modells auf einer Bildschirmausgabe mittels Benutzerinterface-Einrichtungen,
- automatische Bildfusionsverfahren, die insbesondere auf der automatischen Erkennung bestimmter anatomischer Merkmale basieren,
- Bilddaten des generischen Modells, insbesondere digital rekonstruierte Röntgenbilder, und solche aus Computertomographie- oder Kernspintomographie-Bilddatensätzen werden in Deckung gebracht bzw. fusioniert.

Die Fusion des generischen Modells mit Röntgenbilddaten kann also entweder automatisch erfolgen, zum Beispiel durch automatische Erkennung bestimmter anatomischer Merkmale, die für die Fusion entscheidend sind, oder aber manuell, zum Beispiel durch Verschieben, Verdrehen, Stretchen/Verziehen usw. Wenn eine Fusion des generischen Modells mit echten Patienteninformationen mit Hilfe der Akquirierung einer unbestimmten Anzahl von Punktinformationen am Patienten erfolgt (Landmarken), ist es möglich, ein sogenanntes Surface-Matching-Verfahren, also ein computergestütztes Bildanpassungsverfahren zu verwenden, um die Fusion der Bilddaten durchzuführen. Eine Anpassung des generischen Modells kann gemäß einer Ausführungsform der Erfindung so durchgeführt werden, dass neben den zum Beispiel intra-operativ akquirierten Röntgenbilder noch zusätzliche Punkte am Patienten in Form von Landmarken oder zufällig akquirierten Punkten aufgenommen und dazu verwendet werden, die Position des Modells oder dessen Form selbst noch genauer zu erfassen und einzurichten, um damit eine genauere Navigation zu ermöglichen.

Allgemein gesagt, können bei dem erfindungsgemäßen Verfahren die Positionsdaten, die bei der Ermittlung der patientencharakteristischen Erfassungsdaten erhalten werden, insbesondere durch im Navigationssystem registrierte Röntgenbildaufnahmen, dazu verwendet werden, die Registrierung der angepassten Körperstrukturdaten im Navigationssystem vorzunehmen und Behandlungsgeräte bzw. behandlungsunterstützende Geräte in Registrierung zur angepassten Körperstruktur bildlich darzustellen oder einzusetzen. Mit anderen Worten wird der Schritt der Erfassung der Röntgenbilddaten dabei auch gleichzeitig dazu genutzt, die Registrierung von Patient und angepasstem generischen Modell für die Navigation vorzunehmen. Sobald die Daten des Modells mit registrierten Daten, d. h. Daten, die im Raum eindeutig in ihrer Position bestimmt sind, zum Beispiel registrierte Fluoroskopiebilder einer Röntgennavigationssoftware fusioniert werden, oder die Daten des Modells mit Landmarken oder einer Kombination beider Verfahren registriert werden, können diese für die computerassistierte Chirurgie und zum Beispiel für minimal invasive Operationen eingesetzt werden, in dem Instrumente oder Implantate in Relation zu einem fusionierten Modell dargestellt werden.

Das Verfahren gemäß der vorliegenden Erfindung lässt sich sowohl für die Unterstützung chirurgischer Eingriffe anwenden, wobei dem Chirurgen Navigationshilfen auf Bildschirmen bereitgestellt werden, als auch im Rahmen der Strahlentherapie bzw. Radiochirurgie. Die Navigation kann auf einem optischen Tracking oder auf einem magnetischen Tracking beruhen.

Zusammenfassend ist zur obigen Erfindung noch festzustellen, dass sie die Strahlungsbelastung und die Kosten für Schichtbild-Aufnahmeverfahren eliminiert und zumindest minimiert und gegenüber der reinen Röntgennavigation auch noch den Vorteil hat, dass sie dem Chirurgen eine dreidimensionale Navigation und Orientierung ermöglicht. Weitere Vorteile liegen noch darin, dass die Schritte, die zu einer Patientenregistrierung führen, wesentlich unkomplizierter sind und das erfindungsgemäße Verfahren mit wenigen manuellen Schritten zur Patientenregistrierung führen kann. Aufwändige diagnostische Untersuchungen werden vereinfacht und es kann beispielsweise auch die Akquirierung von Punkten/Landmarken am Patienten für die Registrierung überflüssig gemacht werden, wenn bereits kalibrierte Daten (zum Beispiel registrierte Röntgendaten) eingesetzt werden und zusätzliche nützliche Informationen durch Fusion mit dem generischen Modell (zum Beispiel Umwandlung mehrerer zweidimensionaler Informationen zu echten dreidimensionalen Informationen) gewonnen werden.

Die automatische Darstellung der Idealposition von Implantaten oder Instrumenten wird erfindungsgemäß somit mit geringem Aufwand möglich, so dass die Eingriffe schneller, sicherer und weniger invasiv durchführbar sind.

## Patentansprüche

1. Verfahren zur computergestützten medizinischen Navigation bzw. prä-operativen Behandlungsplanung bei dem mittels einer Positionserfassungseinheit die aktuelle Position eines Patienten oder eines Patientenkörperteils im Raum erfasst wird, und bei dem die erfassten Positionsdaten modifizierten Körperstrukturdaten eines generischen Modells zugeordnet werden, um die modifizierten Körperstrukturdaten in Zuordnung zu den Positionsdaten gemeinsam im Rahmen der Behandlungsunterstützung zu verwenden, wobei digital rekonstruierte Röntgenbilder des generischen Modells verwendet werden, wobei die digital rekonstruierten Röntgenbilder mit Röntgenbilddaten des Patienten in Deckung gebracht werden, um die modifizierten Körperstrukturdaten zu erstellen.

2. Verfahren nach Anspruch 1, bei dem die modifizierten Körperstrukturdaten in der Form eines Bilddatensatzes bereitgestellt werden, insbesondere als Schichtbilderfassungs-Datensatz.

3. Verfahren nach Anspruch 1 oder 2, bei dem das generische Modell eine typische bzw. durchschnittliche Körperstruktur umfasst.

4. Verfahren nach einem der Ansprüche 1 bis 3, bei dem das generische Modell ein statistisches Modell der Körperstruktur umfasst, insbesondere basierend auf statistischen Auswertungen einer unbestimmten Anzahl von Bilddatensätzen.

5. Verfahren nach einem der Ansprüche 1 bis 4, bei dem das generische Modell eine Vielzahl von Körperstrukturen gleicher Art umfasst.

6. Verfahren nach einem der Ansprüche 1 bis 5, bei dem das generische Modell ein geometrisches Modell einer Körperstruktur umfasst.

7. Verfahren nach einem der Ansprüche 1 bis 6, bei dem Röntgenbilddaten aus vorab oder während der Behandlung erstellten Röntgenbildern, insbesondere zwei- oder multiplanare Röntgenbilder, verwendet werden.

8. Verfahren nach einem der Ansprüche 1 bis 7, bei dem die Modifizierung der Körperstrukturdaten des generischen Modells durch eine oder mehrere der folgenden Methoden erfolgt:
- manuelle Anpassung mit Hilfe einer Bilddarstellungsunterstützung, insbesondere durch das Versetzen von Punkten und Landmarken oder das Verschieben, Verdrehen, Dehnen, oder Komprimieren des generischen Modells auf einer Bildschirmausgabe mittels Benutzerinterface-Einrichtungen,
- automatische Bildfusionsverfahren, die insbesondere auf der automatischen Erkennung bestimmter anatomischer Merkmale basieren,
- Bilddaten des generischen Modells, insbesondere digital rekonstruierte Röntgenbilder, und solche aus Computertomographie- oder Kernspintomographie-Bilddatensätzen werden in Deckung gebracht bzw. fusioniert,
worauf die angepassten Körperstrukturdaten computergestützt errechnet werden.

## Claims

1. A method for computer-assisted medical navigation and/or pre-operative treatment planning, wherein the current position of a patient or a part of a patient's body is-spatially detected by means of a position detection unit, and wherein said detected positional data are assigned to modified body structure data of a generic model, in order to jointly use said modified body structure data in assignment with said positional data, within the context of assisting the treatment, wherein digitally reconstructed x-ray images of the generic model are used, wherein the digitally reconstructed x-ray images are registered with x-ray image data of the patient in order to produce the modified body structure data.

2. The method as set forth in claim 1, wherein said modified body structure data are provided in the form of an image data set, in particular as a tomographic imaging data set.

3. The method as set forth in claim 1 or 2, wherein said generic model comprises a typical or average body structure.

4. The method as set forth in any one of claims 1 to 3, wherein said generic model includes a statistical model of said body structure, in particular based on statistical evaluations of an indefinite number of image data sets.

5. The method as set forth in any one of claims 1 to 4, wherein said generic model includes a multitude of body structures of the same type.

6. The method as set forth in any one of claims 1 to 5, wherein said generic model comprises a geometric model of a body structure.

7. The method as set forth in any one of claims 1 to 6, wherein x-ray image data from x-ray images produced before or during treatment are used, in particular bi-planar or multiplanar x-ray images.

8. The method as set forth in any one of claims 1 to 7, wherein said body structure data of said generic model are modified using one or more of the following methods:
- manually adapting with the assistance of image representation, in particular by offsetting points and landmarks or by shifting, rotating, stretching or compressing said generic model on a screen output by means of user-interface means;
- automatic image fusion methods, in particular based on automatically identifying particular anatomical features;
- registering and/or fusing image data of said generic model, in particular digitally reconstructed x-ray images, and the same from computer tomography or nuclear spin tomography image data sets,
after which the adapted body structure data are calculated by computer-assistance.

## Revendications

1. Procédé de navigation médicale ou de planification d'un traitement pré-opératoire assistées par ordinateur, dans lequel on détecte, au moyen d'une unité de détection de position, la position actuelle dans l'espace d'un patient ou d'une partie du corps d'un patient, et dans lequel les données de position détectées sont associées à des données de structure corporelle modifiées d'un modèle générique, afin d'utiliser ensemble les données de structure corporelle modifiées en correspondance avec les données de position dans le cadre de l'assistance au traitement, des radiographies reconstruites de manière numérique du modèle générique étant utilisées, les radiographies reconstruites de manière numérique étant amenées en coïncidence avec des données radiographiques du patient, afin d'établir les données de structure corporelle modifiées.

2. Procédé selon la revendication 1, dans lequel les données de structure corporelle modifiées sont préparées sous la forme d'un ensemble de données graphiques, en particulier sous la forme d'un ensemble de données tomographiques.

3. Procédé selon la revendication 1 ou 2 dans lequel le modèle générique comprend une structure corporelle typique ou moyenne.

4. Procédé selon l'une des revendications 1 à 3, dans lequel le modèle générique comprend un modèle statistique de la structure corporelle, basé en particulier sur des études statistiques d'un nombre indéterminé d'ensembles de données graphiques.

5. Procédé selon l'une des revendications 1 à 4, dans lequel le modèle générique comprend un grand nombre de structures corporelles de même type.

6. Procédé selon l'une des revendications 1 à 5, dans lequel le modèle générique comprend un modèle géométrique d'une structure corporelle.

7. Procédé selon l'une des revendications 1 à 6, dans lequel on utilise des données radiographiques provenant de radiographies établies avant ou pendant le traitement, en particulier de radiographies biplanaires ou multiplanaires.

8. Procédé selon l'une des revendications 1 à 7, dans lequel la modification des données de structure corporelle du modèle générique s'effectue par une ou plusieurs des méthodes suivantes :
- adaptation manuelle à l'aide d'une assistance graphique, en particulier par le déplacement de points et de repères ou la translation, la rotation, l'extension ou la compression du modèle générique sur un terminal à écran au moyen de dispositifs d'interface pour utilisateur,
- procédés automatiques de fusion d'images qui se basent en particulier sur la reconnaissance automatique de caractéristiques anatomiques déterminées,
- des données graphiques du modèle générique, en particulier radiographies reconstruites de manière numérique, et des données provenant d'ensembles de données graphiques de la tomographie assistée par ordinateur ou à résonance magnétique nucléaire sont amenées en coïncidence ou fusionnées,
après quoi les données de structure corporelle adaptées sont calculées de manière assistée par ordinateur.
